# EUROPÄISCHE PATENTSCHRIFT

(11) **EP 4 081 137 B1**
(45) Veröffentlichungstag und Bekanntmachung des Hinweises auf die Patenterteilung: **03.05.2023**
(21) Anmeldenummer: 21717014.1
(22) Anmeldetag: 01.04.2021
(51) Int. Cl.: A61B 17/00, A61B 90/00, A61B 17/16, A61C 1/00

(54) **ADAPTIVER WERKZEUGBETRIEB**
ADAPTIVE TOOL OPERATION
FONCTIONNEMENT D'INSTRUMENT ADAPTATIF

(30) Priorität: 09.04.2020 DE 102020109932
(43) Veröffentlichungstag der Anmeldung: 02.11.2022
(73) Patentinhaber: Aesculap AG, 78532 Tuttlingen (DE)
(72) Erfinder: HÖGERLE, Roland-Alois, 78532 Tuttlingen (DE)
(74) Vertreter: Winter, Brandl - Partnerschaft mbB
(86) Internationale Anmeldenummer: PCT/EP2021/058602
(87) Internationale Veröffentlichungsnummer: WO 2021/204664

(56) Entgegenhaltungen:
- EP-A2- 0 890 343
- EP-B1- 0 890 343
- WO-A1-2018/194909
- DE-A1-102018 130 376

## Beschreibung

Die Erfindung betrifft eine medizinische Vorrichtung, insbesondere ein chirurgisches Motorsystem.

### Hintergrund

Hintergrund der Erfindung ist das Bereitstellen eines automatisierten und kontrollierten Betriebs einer medizinischen Vorrichtung. Hierunter fallen chirurgische Motorensysteme, wie Handstücke und Handfräsgeräte. Mitunter haben die chirurgischen Motorensysteme ein Handstück und eine daran anbringbare oder angebrachte Distanzhülse, mit deren Hilfe größere Distanzen überbrückt werden können, ohne das Gerät näher an den Patienten heran führen zu müssen. Im Laufe einer Verwendung leidet das Gerät unter Verschleiß, der mehrere Ursprünge haben kann. Hierunter fallen Schmiermittelabnutzung und Abnutzung der in einem Antriebsstrang des chirurgischen Motorensystems enthaltene Elemente.

Es kann ein Bedarf bestehen zum Bereitstellen von Konzepten, die den Verschleiß der medizinischen Vorrichtung mitberücksichtigen.

EP 0 890 343 A2 offenbart ein medizinisches Behandlungssystem aufweisend eine Steuereinheit und eine mit der Steuerung verbindbare Versorgungsleitung, über die die Steuereinheit den Betrieb eines Behandlungswerkzeugs steuert. Relevanter Stand der Technik ist ferner in WO 2018/194909 A1 und DE 10 2018 130376 A1 bekannt.

### Kurzbeschreibung der Erfindung

Es ist also die Aufgabe der Erfindung, eine medizinische Vorrichtung dazu bereitzustellen, das einen sicheren und kontrollierten Betrieb auch unter Verschleiß bereitstellt.

Diese Aufgabe wird erfindungsgemäß dadurch gelöst, dass eine medizinische Vorrichtung, insbesondere ein chirurgisches Motorsystem, bereitgestellt wird. Die medizinische Vorrichtung hat ein Steuergerät. Das Steuergerät ist ausgebildet, einen Sollstrombedarf gemäß Anforderungen eines Antriebs der medizinischen Vorrichtung zu speichern. Das Steuergerät ist ferner ausgebildet, einen minimalen Strombedarf entsprechend einer minimalen Anforderung des Antriebs der medizinischen Vorrichtung während eines aktuellen Betriebs zu bestimmen. Ferner ist das Steuergerät ausgebildet, basierend auf dem minimalen Strombedarf, einen für eine Strombegrenzung vorgesehenen Maximalwert des Sollstrombedarfs für den aktuellen Betrieb der medizinischen Vorrichtung anzupassen.

Somit lässt sich ein sicherer und kontrollierter Betrieb unter Verschleiß bereitstellen.

Die hierin genannten Anforderungen können Anforderungen im Sinne von Drehmomentanforderungen sein. Hierbei kann über ein Fußpedal der medizinischen Vorrichtung ein Drehmoment von dem Antrieb der medizinischen Vorrichtung angefordert sein. Die Drehmomentanforderung kann direkt proportional zu dem Strombedarf sein. Der Strombedarf kann ein Gesamtstrombedarf der medizinischen Vorrichtung sein, der beim Anschluss an eine Energieversorgung bei Verwendung verbraucht wird. Hierbei kann der Strombedarf an eine Last im Sinne einer Drehmomentanforderung und zu bearbeitendem Material, zum Beispiel Knochen, angepasst sein.

Das Steuergerät kann ausgebildet sein, den minimalen Strombedarf während dem aktuellen Betrieb kontinuierlich zu messen bzw. zu bestimmen. Das Steuergerät kann ausgebildet sein, den Maximalwert des Sollstrombedarfs bei einer Veränderung des minimalen Strombedarfs entsprechend anzupassen.

Somit kann der Maximalwert adaptiv angepasst werden.

Das Steuergerät kann sich innerhalb eines Handstücks der medizinischen Vorrichtung befinden. Zum Beispiel in einem dafür vorgesehenen Griffstück, das ausgebildet ist, von einem Nutzer umfasst zu werden.

Der minimale Strombedarf kann einem Leerlaufbetrieb der medizinischen Vorrichtung während des aktuellen Betriebs entsprechen. Somit kann auch ein Minimalwert des Sollstrombedarfs einem Leerlaufbetrieb der medizinischen Vorrichtung während eines ersten Betriebs oder während einer ersten Inbetriebnahme oder einer ersten Messreihe der medizinischen Vorrichtung entsprechen.

In anderen Worten, der Minimalwert des Sollstrombedarfs kann dem Leerlaufbetrieb der medizinischen Vorrichtung zeitlich vor dem aktuellen Betrieb entsprechen.

Somit können unterschiedliche Zeitreihen verglichen werden und ein Maximalstrombedarf im Sinne eines maximalen Drehmomentbedarfs angepasst werden.

Der aktuelle Betrieb kann ein Betrieb nach ein oder mehrmaliger Benutzung der medizinischen Vorrichtung sein. Der aktuelle Betrieb kann einer aktuellen Verwendung, zum Beispiel bei Benutzung und aktiver Energieversorgung der medizinischen Vorrichtung, entsprechen.

Der Sollstrombedarf kann einer dedizierten Kombination aus Drehmoment beeinflussenden Elementen eines Antriebsstranges der medizinischen Vorrichtung, zum Beispiel Motor/Getriebe/Welle/Kugellager/Werkzeug zugeordnet sein. Diese Kombination kann den Sollstrombedarf festlegen. Der Sollstrombedarf kann in Form einer Kurve, zum Beispiel ein Kurvenverlauf, sein oder dargestellt sein.

Hierdurch lässt sich für jede Art der Verwendung ein angepasster Strombedarf einstellen bzw. vorgeben.

Der gespeicherte Sollstrombedarf kann als Daten in einem Speicher des Steuergeräts gespeichert sein. Das Steuergerät kann hierfür einen separaten Speicher außerhalb der medizinischen Vorrichtung bzw. des Handstücks aufweisen. Ebenfalls kann der Speicher innerhalb der medizinischen Vorrichtung bzw. des Handstücks vorhanden sein. Der Speicher kann auch Teil eines integrierten Steuergeräts im Sinne einer Platine sein. Der Speicher kann in Form eines EEPROM bereitgestellt sein und kann einfach in das Handstück untergebracht sein.

Das Steuergerät kann ausgebildet sein, wenn der minimale Strombedarf einen Schwellenwert überschreitet, ein eine Information über einen Verschleiß der medizinischen Vorrichtung enthaltendes Signal auszulösen, das für einen Benutzer der medizinischen Vorrichtung bestimmt ist. Das Signal kann an eine Anzeigevorrichtung oder einen Speicher weitergegeben bzw. hineingeschrieben werden. Hierdurch lässt sich die Information des Signals entweder an den Benutzer weitergeben oder in dem Speicher zur weiteren Verwendung loggen.

Ein Verfahren zum Betreiben einer medizinischen Vorrichtung, vorzugsweise wie oben beschrieben, umfasst Speichern, durch ein Steuergerät der medizinischen Vorrichtung, eines Sollstrombedarfs gemäß Anforderungen eines Antriebs der medizinischen Vorrichtung. Das Verfahren umfasst ferner Bestimmen, durch das Steuergerät, eines minimalen Strombedarfs entsprechend einer minimalen Anforderung des Antriebs der medizinischen Vorrichtung während eines aktuellen Betriebs. Das Verfahren umfasst außerdem Anpassen, durch das Steuergerät, basierend auf dem minimalen Strombedarf, eines für eine Strombegrenzung vorgesehenen Maximalwerts des Sollstrombedarfs für den aktuellen Betrieb der medizinischen Vorrichtung. Das Verfahren nicht unter den Wortlaut der Ansprüche, werden aber als das Verständnis der Erfindung erleichternd angesehen.

Somit lässt sich ein sicherer und kontrollierter Betrieb unter Verschleiß bereitstellen.

Der Sollstrombedarf kann einem Ab Werk Zustand der medizinischen Vorrichtung entsprechen. Der gespeicherte Sollstrombedarf kann während eines ersten Betriebs der medizinischen Vorrichtung gemessen bzw. bestimmt worden sein.

Das heißt, die medizinische Vorrichtung wird mit einem Steuergerät ausgestattet, die Daten bezüglich des Sollstrombedarfs enthält bzw. gespeichert hat. Hiermit kann die medizinische Vorrichtung an einen Kunden ausgeliefert werden, der mit einer geregelten medizinischen Vorrichtung ausgerüstet wird. Die Daten können ferner erhoben werden. Hierzu kann ein Server bereitgestellt werden, der Informationen über Strombedarf und Sollstrombedarf speichert. Diese Daten können zusammen mit von Nutzern gelieferten Verschleißinformationen bereitgestellt sein, worauf Rückschlüsse auf Verwendung und Zustand der medizinischen Vorrichtung getroffen werden können.

Mit anderen Worten betrifft die Erfindung die Ermittlung relevanter Daten und Datenbereitstellung, zum Beispiel einer künstlichen Intelligenz, zum sicheren und automatisch kontrollierten Betrieb von Werkzeugen und Antrieben. Hierbei kann eine Datengenerierung ohne zusätzliche Sensoren erfolgen, ausschließlich basierend auf bereits vorhandenen Daten im Steuergerät zum Motorbetrieb.

In einem oder mehreren Ausführungsformen kann ein Gesamtantriebsstrom entlang der Zeit gemessen/bestimmt bzw. abgespeichert sein. Die Methodik hierfür kann wie folgt vorgesehen sein.

Zuerst kann eine Ermittlung des Gesamtantriebsstromverlaufs "Referenzkurve" (insbesondere unter Laborbedingungen) durchgeführt werden. Jede individuelle Kombination (Motor/Getriebe/Werkzeug) im idealen Neuzustand bei optimaler Tribologie (Ölart/Ölmenge) bis zur Belastung des jeweiligen Werkzeugs zum maximalen Abtrag ohne Werkzeugüberlastung kann hierfür berücksichtigt sein.

Danach kann eine Festlegung des maximalen Gesamtantriebsstroms (Imax opt= Mmaxopt: Maximalwert der Gesamtantriebsstromkurve = Strombegrenzung, dieser Wert entspricht dem maximal zulässigen Drehmoment an der Werkzeugschneide des jeweiligen Werkzeugs) durchgeführt werden.

Hiernach kann eine Begrenzung des Gesamtantriebsstromes (bei Imax opt) festgelegt werden. Dadurch kann eine Begrenzung des maximalen Drehmoments an einer Werkzeugschneide für die individuelle Kombination durch das Steuergerät festgelegt werden. Diese Drehmomentbegrenzung der jeweiligen Werkzeuge stimmt (bei diesem Verfahren) nur beim Betrieb mit idealen Antrieben (neuwertig, ideale Schmierung).

Zum Beispiel muss bei zunehmendem Verschleiß des Motors und des Getriebes sowie ungeeigneter Schmiermenge / Schmiermittel zum maximalen Gesamtantriebsstrom (Imax opt) ein individueller Korrekturwert (Icor) addiert werden. Der zunehmende Verschleiß sowie ungeeignete Schmierung erhöhen die Betriebsdrehmomente des Systems und reduzieren das Drehmoment an der Werkzeugschneide.

Ferner kann eine Methodik zur Ermittlung des Verschleißkorrekturwertes vorgesehen sein. Die Minima der Gesamtantriebsstromkurve (Isys) können dem Strombedarf (Drehmomentbedarf) des Gesamtantriebssystems ohne Knochenkontakt entsprechen (Fräser läuft bei der jeweiligen Drehzahl (vorgegeben durch z.B. einer mit der medizinischen Vorrichtung gekoppelte die Drehmomentanforderung bereitstellende Pedalstellung) ohne Kontakt zum Knochen, Drehmoment an der Schneide =0 Nm).

Während der gesamten Opersation können die Minima der Gesamtantriebsstromkurve (Isysist) ermittelt werden. Eine Erhöhung der Minimawerte (Isysist) im Vergleich zur Referenzkurve (Isysopt) kann ein Maß für den Verschleiß und den Schmierzustand des Antriebssystems sein. Die Differenz der jeweiligen Minimawertmessung (Isysist) zum Referenzminimawert (Isysopt) wird zur Strombegrenzung = Maximalwert der Gesamtantriebsstromkurve addiert (Imaxist(t) = Imaxopt(t) + Icor(t)).

Übersteigt darüber hinaus die Differenz des aktuell im Prozess gemessenen bzw. bestimmten Minimums einen vorgegeben Wert (Icormax), ist das zulässige Verschleißmaß erreicht. Dies kann dann dokumentiert und in den weiteren Informationsfluss einfließen (predictiv maintenance, englisch für prädiktive Wartung).

Ferner können weitere Erkenntnisse aus der fortlaufenden Messung des Leerlaufstroms (Isysist) und zur Datenbereitstellung für eine künstliche Intelligenz bereitgestellt sein.

Zum Beispiel kann nur bei einem optimalen System (Neuzustand, Schmierung) der Verlauf des Leerlaufstroms (Isysist) nahezu konstant sein.

Als weiteres Beispiel kann bei neuwertigen Systemen die "überölt" sind, der Wert von einem zu hohen Leerlaufstrom (Isysist) im Laufe der Operation sinken.

Des Weiteren kann bei einem unzureichend geölten System der Leerlaufstrom (Isysist) im Verlauf der Operation signifikant ansteigen.

Ebenso können aus dem Stromanstieg des Leerlaufstroms (Isysist) Rückschlüsse auf Art und Form des Werkzeuges ermöglicht werden.

Schließlich kann von weiteren Erkenntnissen und Zusammenhängen mit zunehmender Erfahrung dieser Messtechnik ausgegangen werden.

Es ist dem Fachmann klar, dass die hierin dargelegten Erklärungen unter Verwendung von Hardwareschaltungen, Softwaremitteln oder einer Kombination davon implementiert sein/werden können. Die Softwaremittel können im Zusammenhang stehen mit programmierten Mikroprozessoren oder einem allgemeinen Computer, einer ASIC (Englisch: Application Specific Integrated Circuit; zu Deutsch: anwendungsspezifische integrierte Schaltung) und/oder DSPs (Englisch: Digital Signal Processors; zu Deutsch: digitale Signalprozessoren).

Beispielsweise kann die medizinische Vorrichtung, insbesondere das Steuergerät, teilweise als ein Computer, eine Logikschaltung, ein FPGA (Field Programmable Gate Array; zu Deutsch: im Feld programmierbare Logik-Gatter-Anordnung), ein Prozessor (beispielsweise umfassend einen Mikroprozessor, einen Mikrocontroller (µC) oder einen Vektorprozessor)/Core (zu Deutsch: Hauptspeicher, kann in dem Prozessor integriert sein beziehungsweise von dem Prozessor verwendet werden)/CPU (Englisch: Central Processing Unit; zu Deutsch: zentrale Prozessoreinheit; wobei mehrere Prozessorkerne möglich sind), eine FPU (Englisch: Floating Point Unit; zu Deutsch: Gleitkommaprozessoreinheit), eine NPU (Englisch: Numeric Processing Unit; zu Deutsch: Numerische Prozessoreinheit), eine ALU (Englisch: Arithmetic Logical Unit; zu Deutsch: arithmetisch-logische Einheit), ein Koprozessor (zusätzlicher Mikroprozessor zur Unterstützung eines Hauptprozessors (CPU)), eine GPGPU (Englisch: General Purpose Computation on Graphics Processing Unit; zu Deutsch: Allzweck-Berechnung auf Grafikprozessoreinheit(en)), ein Parallelrechner (zum gleichzeitigen Ausführen, unter anderem auf mehreren Hauptprozessoren und/oder Grafikprozessoren, von Rechenoperationen) oder ein DSP realisiert sein.

Es ist dem Fachmann zu dem klar, dass auch dann wenn die hierin beschriebenen Details in Bezug auf ein Verfahren beschrieben werden, diese Details auch in einer geeigneten Vorrichtung, einem Computerprozessor oder einem mit einem Prozessor verbundenen Speicher realisiert sein können, wobei der Speicher mit einem oder mehreren Programmen versehen ist, die das Verfahren durchführen, wenn sie durch den Prozessor ausgeführt werden. Hierbei können Verfahren wie Swapping (zu Deutsch: Umlagerung) und Paging (zu Deutsch: Kachelverwaltung) verwendet werden.

Auch wenn einige der voranstehend beschriebenen Aspekte in Bezug auf die medizinische Vorrichtung beschrieben wurden, so können diese Aspekte auch auf das Verfahren zutreffen. Genauso können die voranstehend in Bezug auf das Verfahren beschriebenen Aspekte in entsprechender Weise auf die Vorrichtung zutreffen.

Heißt es vorliegend, dass eine Komponente mit einer anderen Komponente "verbunden ist", damit "in Verbindung steht" oder "darauf zugreift", kann dies heißen, dass sie damit unmittelbar verbunden ist oder auf diese unmittelbar zugreift; hierbei ist aber anzumerken, dass eine weitere Komponente dazwischenliegen kann. Heißt es andererseits, dass eine Komponente mit einer anderen Komponente "unmittelbar verbunden" ist oder "unmittelbar darauf zugreift", ist darunter zu verstehen, dass dazwischen keine weiteren Komponenten vorhanden sind.

### Kurzbeschreibung der Figuren

Die Erfindung wird nachfolgend mit Hilfe von Zeichnungen erläutert. Es zeigen:
- Figur 1: eine schematische Darstellung eines aktuellen Antriebsstroms und einer Referenzstromkurve; und
- Figur 2: eine schematische Darstellung eines aktuellen Antriebsstroms und einer Referenzstromkurve.

Die Figuren sind lediglich schematischer Natur und dienen ausschließlich dem Verständnis der Erfindung. Die gleichen Elemente sind mit denselben Bezugszeichen versehen. Die Merkmale der einzelnen Ausführungsformen können untereinander ausgetauscht werden.

Darüber hinaus können hier räumlich relative Begriffe, wie etwa "darunter befindlich", "unter", "untere(r)"/"unteres", "darüber befindlich", "obere(r)"/"oberes", "links", "linke(r)/linkes", "rechts", "rechte(r)/rechtes" und dergleichen, zur einfachen Beschreibung der Beziehung eines Elements oder einer Struktur zu einem oder mehreren anderen Elementen oder Strukturen verwendet werden, die in den Figuren dargestellt sind. Die räumlich relativen Begriffe sollen zusätzlich zu der in den Figuren dargestellten Orientierung andere Orientierungen des in Gebrauch oder in Betrieb befindlichen Bauelements umfassen. Das Bauelement kann anders ausgerichtet werden (um 90 Grad gedreht oder in einer anderen Orientierung), und die räumlich relativen Deskriptoren, die hier verwendet werden, können ebenso entsprechend interpretiert werden.

### Figurenbeschreibung

Die medizinische Vorrichtung und das Verfahren werden nun anhand von Ausführungsformen beschrieben. Im Folgenden wird das anhand von Graphen geschehen.

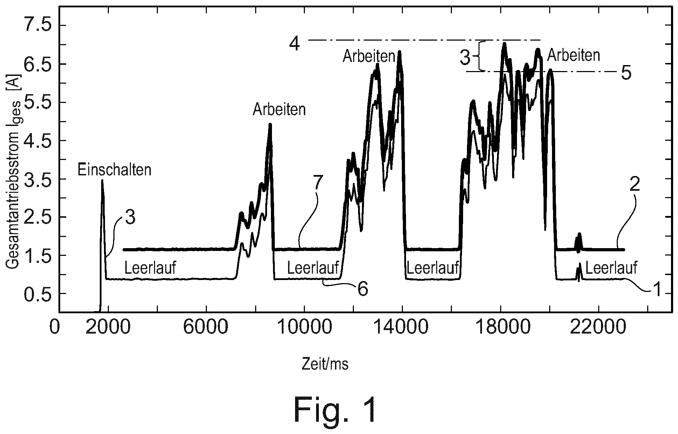

Fig. 1 zeigt eine schematische Darstellung eines aktuellen Antriebsstroms und einer Referenzstromkurve entsprechend dem Antriebsstrom, der einer optimalen medizinischen Vorrichtung entspricht. Die optimale medizinische Vorrichtung ist so zu verstehen, dass ein Verschleiß der Elemente der medizinischen Vorrichtung minimiert ist und eine optimale Schmierung des Antriebsstrangs der medizinischen Vorrichtung vorliegt.

In Fig. 1 sind zwei Kurvenverläufe zu sehen, die eine bezeichnet einen Sollstrombedarf im Sinne einer Referenzkurve 1 und die andere bezeichnet einen aktuellen Strombedarf 2, der vorliegt bei einem anzunehmenden aktuellen Betrieb der medizinischen Vorrichtung, wie sie hierin beschrieben wurde. Auf der Ordinate ist der Gesamtantriebsstrom in Ampere angegeben und auf der Abszisse die Zeit in Millisekunden.

Im Folgenden wird auf die Referenzkurve 1 eingegangen. Von links nach rechts sind die einzelnen Kurvenveränderungen dergestalt zu sehen, dass in einem Einschaltzeitpunkt der Strom spitzenartig ansteigt und wieder abfällt bis er einen Leerlaufstrom erreicht, der knapp unterhalb von 1 A liegt. Weiter rechts davon geht die medizinische Vorrichtung in einen Arbeitsmodus über, wodurch der Strombedarf ansteigt. Nach Beendigung des Arbeitsschrittes geht die Stromkurve in den Leerlauf über. Der Leerlauf bezeichnet insbesondere den minimalen Sollstrombedarf 6. Nach dem Leerlauf geht die medizinische Vorrichtung bei Bedarf in einen Arbeitsmodus über, der höher als der erste Arbeitsmodus ist und somit die Sollstromkurve (Referenzkurve) 1 wiederum ansteigt. Durch die unterschiedlichen Anforderungen während des Arbeitsschrittes verläuft die Sollstromkurve 1 nicht linear sondern hat lediglich eine Tendenz entsprechend steigenden Gesamtantriebsstroms. Nach Beendigung des Arbeitsschrittes geht die medizinische Vorrichtung wieder in den Leerlauf über, also den minimalen Sollstrombedarf 6. Hiernach erfolgt wiederum ein Arbeitsschritt, der wie der Stromverlauf zeigt intensiver und länger abläuft und somit einen höheren Sollstrombedarf benötigt. Hierbei erreicht er ein Maximum 5, das dem maximalen Sollstrombedarf 5 entspricht. Nach Beendigung der Arbeit und bei weiterem Anschluss an eine Energieversorgung geht die medizinische Vorrichtung wieder in den Leerlaufbetrieb und somit bedarf es lediglich dem minimalen Sollstrombedarf 6.

Im Vergleich zur Referenzkurve 1 ist der aktuelle Strombedarf 2 hier durch einen Absatz, nämlich einem Korrekturwert 3 in Ordinatenrichtung verschoben. Hierdurch ergibt sich die Verschiebung der Kurve im Sinne der Fig. 1 nach oben. Der so eingestellte maximale aktuelle Strombedarf erhöht sich damit auf den maximalen Strombedarf 4, der sich von dem maximalen Sollstrombedarf 5 durch den Korrekturwert 3 unterscheidet. Der Korrekturwert 3 ergibt sich aus dem Unterschied der Leerlaufmodi im aktuellen Zustand verglichen zu dem optimalen Zustand gemäß der Sollstrombedarfskurve 1. Hierbei werden der minimale aktuelle Strombedarf 7 und der minimale Sollstrombedarf 6 miteinander verglichen, bzw. lediglich eine Differenz gebildet, die auf den maximalen Sollstrombedarf 5 aufaddiert wird. Hierdurch ergibt sich der maximale aktuelle Strombedarf 4. Des Weiteren sei darauf hingewiesen, dass sich die jeweiligen Arbeitsschritte hier gleichen, nur dass es sich bei der aktuellen Strombedarfskurve 2 um eine bereits in Verwendung befindliche medizinische Vorrichtung handelt.

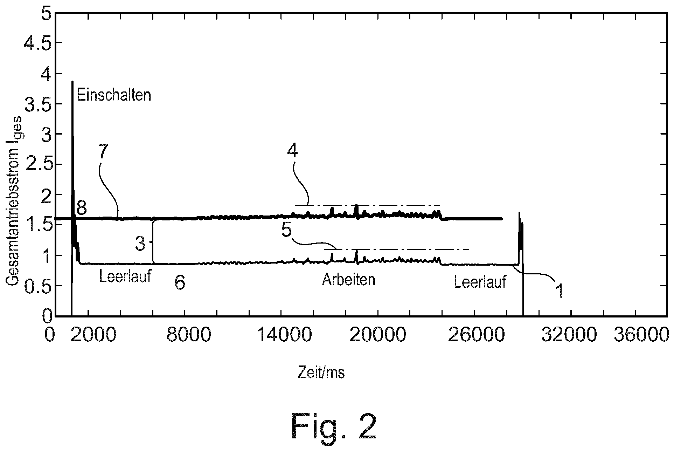

Weitere Einzelheiten und Aspekte sind in Verbindung mit den vor- oder nachstehend beschriebenen Ausführungsformen erwähnt. Die in Fig. 1 gezeigte Ausführungsform kann ein oder mehrere optionale zusätzliche Merkmale aufweisen, die einem oder mehreren Aspekten entsprechen, die in Verbindung mit dem vorgeschlagenen Konzept oder nachstehend in Bezug auf Fig. 2 beschriebenen Ausführungsformen erwähnt sind.

Fig. 2 zeigt eine schematische Darstellung eines aktuellen Antriebsstroms und einer Referenzstromkurve gemäß einem Antriebsstrom. Der Unterschied zwischen Fig. 1 und Fig. 2 liegt darin, dass in Fig. 1 ein Fräser mit einem größeren Durchmesser, nämlich 6 mm, verwendet wurde, wohingegen in Fig. 2 ein Fräser mit einem kleineren Durchmesser, nämlich 1 mm, verwendet wurde. Der Hauptunterschied in der Betrachtung der Fign. 1 und 2 liegt in der Tendenz der Stromverläufe, wohingegen bei Fig. 1 starke Anstiege gezeigt sind, sind in Fig. 2 weniger starke Anstiege vorhanden.

Ebenfalls sind in Fig. 2 der Gesamtantriebsstrom über der Zeit in ms angezeigt. In einem Einschaltzustand der medizinischen Vorrichtung steigt der Strom stark an, fast bis zu 4 A. Danach sinkt der Strom wieder auf einen minimalen Sollstrombedarf, gemäß einem Leerlaufzustand der medizinischen Vorrichtung. Nach dem Leerlaufmodus beginnt der Arbeitsmodus, der durch die Rippel im Kurvenverlauf gezeigt wird. Die Rippel umfassen einen maximalen Sollstrombedarf 5, wohingegen der Kurvenverlauf im Leerlaufbetrieb einen minimalen Sollstrombedarf umfasst. Hierdurch kann beim Arbeiten eine Strombegrenzung gemäß dem maximalen Sollstrombedarf festgelegt werden. Nach Beendigen des Arbeitsmodus geht die medizinische Vorrichtung wieder in den Leerlaufmodus und nimmt den minimalen Sollstrombedarf 6 an. Dieses Vorgehen entspricht dem Vorgehen aus Fig. 1. Ebenfalls ist zu sehen, dass mittels des Korrekturwerts 3 nicht der maximale Sollstrombedarf gedeckt wird sondern der maximale aktuelle Strombedarf 4. Der maximale Strombedarf 4 ergibt sich demnach aus einer Addition des Korrekturwerts 3 und des maximalen Sollstrombedarfs 5.

Im Vergleich zu Fig. 1 wird in Fig. 2 außerdem ein Korrekturwertmaximum 8 gezeigt, der eine Grenze für den Korrekturwert 3 bilden soll. Bei Übersteigen des Korrekturwerts 3 über einen Schwellenwert, welcher durch das Korrekturwertmaximum 8 gegeben ist, können verschiedene Ereignisse stattfinden. Diese Ereignisse können sein, das Weiterleiten einer Nachricht an eine übergeordnete Instanz oder das Mitteilen dieser Information an einen Benutzer. Hierüber kann festgestellt werden, dass zu viel Verschleiß oder eine Über- bzw. Unterölung der medizinischen Vorrichtung bzw. der Elemente davon vorliegt.

### Bezugszeichenliste

- 1: Sollstrombedarf (Referenzkurve)
- 2: aktueller Strombedarf
- 3: Korrekturwert (Icor)
- 4: maximaler aktueller Strombedarf (Imaxist)
- 5: maximaler Sollstrombedarf (Imaxopt)
- 6: minimaler Sollstrombedarf (Isysopt)
- 7: minimaler aktueller Strombedarf (Isysist)
- 8: Korrekturwertmaximum (Icor)

## Patentansprüche

1. Medizinische Vorrichtung mit einem Steuergerät, wobei das Steuergerät ausgebildet ist, einen Sollstrombedarf (1) gemäß Anforderungen eines Antriebs der medizinischen Vorrichtung zu speichern, und einen minimalen Strombedarf (7) entsprechend einer minimalen Anforderung des Antriebs der medizinischen Vorrichtung während eines aktuellen Betriebs zu bestimmen, **dadurch gekennzeichnet, dass** das Steuergerät auch ausgebildet ist, basierend auf dem minimalen Strombedarf (7), einen für eine Strombegrenzung vorgesehenen Maximalwert (4) des Sollstrombedarfs (1) für den aktuellen Betrieb der medizinischen Vorrichtung anzupassen.

2. Die medizinische Vorrichtung nach Anspruch 1, **dadurch gekennzeichnet, dass** das Steuergerät ausgebildet ist, den minimalen Strombedarf (7) während des aktuellen Betriebs kontinuierlich zu messen und den Maximalwert (4) des Sollstrombedarfs (1) bei einer Veränderung des minimalen Strombedarfs (7) entsprechend anzupassen.

3. Medizinische Vorrichtung nach Anspruch 1 oder 2, **dadurch gekennzeichnet, dass** der minimale Strombedarf (7) einem Leerlaufbetrieb der medizinischen Vorrichtung während des aktuellen Betriebs entspricht.

4. Medizinische Vorrichtung nach einem der vorhergehenden Ansprüche, **dadurch gekennzeichnet, dass** ein Minimalwert (6) des Sollstrombedarfs (1) einem Leerlaufbetrieb der medizinischen Vorrichtung zeitlich vor dem aktuellen Betrieb entspricht.

5. Medizinische Vorrichtung nach einem der vorhergehenden Ansprüche, **dadurch gekennzeichnet, dass** der aktuelle Betrieb ein Betrieb nach ein oder mehrmaliger Benutzung der medizinischen Vorrichtung ist.

6. Medizinische Vorrichtung nach einem der vorhergehenden Ansprüche, **dadurch gekennzeichnet, dass** der Sollstrombedarf (1) einer dedizierten Kombination aus Drehmoment beeinflussenden Elementen eines Antriebsstranges der medizinischen Vorrichtung zugeordnet ist.

7. Medizinische Vorrichtung nach einem der vorhergehenden Ansprüche, **dadurch gekennzeichnet, dass** der gespeicherte Sollstrombedarf (1) als Daten in einem Speicher des Steuergeräts gespeichert ist.

8. Medizinische Vorrichtung nach einem der vorhergehenden Ansprüche, **dadurch gekennzeichnet, dass** das Steuergerät ausgebildet ist, wenn der minimale Strombedarf (7) einen Schwellenwert (8) überschreitet, ein eine Information über einen Verschleiß der medizinischen Vorrichtung enthaltendes Signal auszulösen, das für einen Benutzer der medizinischen Vorrichtung bestimmt ist.

## Claims

1. A medical device comprising a control device, wherein the control device is configured to store a target current demand (1) according to requirements of a drive of the medical device, and to determine a minimum current demand (7) according to a minimum requirement of the drive of the medical device during a current operation, **characterized in that** the control device is also configured, based on the minimum current demand (7), to adjust a maximum value (4) of the target current demand (1) provided to limit the current for the current operation of the medical device.

2. The medical device according to claim 1, **characterized in that** the control device is configured to continuously measure the minimum current demand (7) during the current operation and to adapt the maximum value (4) of the target current demand (1) accordingly in case the minimum current demand (7) changes.

3. The medical device according to claim 1 or 2, **characterized in that** the minimum current demand (7) corresponds to a no-load operation of the medical device during the current operation.

4. The medical device according to one of the preceding claims, **characterized in that** a minimum value (6) of the target current demand (1) corresponds to a no-load operation of the medical device in time before the current operation.

5. The medical device according to one of the preceding claims, **characterized in that** the current operation is an operation after one or more times of use of the medical device.

6. The medical device according to one of the preceding claims, **characterized in that** the target current demand (1) is associated with a dedicated combination of torque-effecting elements of a drive train of the medical device.

7. The medical device according to one of the preceding claims, **characterized in that** the stored target current demand (1) is stored as data in a memory of the control device.

8. The medical device according to one of the preceding claims, **characterized in that** the control device is configured, when the minimum current demand (7) exceeds a threshold value (8), to trigger a signal containing information about wear of the medical device, which is intended for a user of the medical device.

## Revendications

1. Dispositif médical comportant un appareil de commande, dans lequel l'appareil de commande est configuré pour stocker un besoin en courant de consigne (1) selon les exigences d'un entraînement du dispositif médical et pour déterminer un besoin en courant minimal (7) en fonction d'une exigence minimale de l'entraînement du dispositif médical pendant un fonctionnement en cours, **caractérisé en ce que** l'appareil de commande est également configuré pour adapter, sur la base du besoin en courant minimal (7), une valeur maximale (4) prévue pour une limitation de courant du besoin en courant de consigne (1) pour le fonctionnement en cours du dispositif médical.

2. Dispositif médical selon la revendication 1, **caractérisé en ce que** l'appareil de commande est configuré pour mesurer en continu le besoin en courant minimal (7) pendant le fonctionnement en cours et pour adapter en conséquence la valeur maximale (4) du besoin en courant de consigne (1) lors d'une modification du besoin en courant minimal (7).

3. Dispositif médical selon la revendication 1 ou 2, **caractérisé en ce que** le besoin en courant minimal (7) correspond à un fonctionnement à vide du dispositif médical pendant le fonctionnement en cours.

4. Dispositif médical selon l'une des revendications précédentes, **caractérisé en ce qu'**une valeur minimale (6) du besoin en courant de consigne (1) correspond à un fonctionnement à vide du dispositif médical dans le temps avant le fonctionnement en cours.

5. Dispositif médical selon l'une des revendications précédentes, **caractérisé en ce que** le fonctionnement en cours est un fonctionnement après une ou plusieurs utilisations du dispositif médical.

6. Dispositif médical selon l'une des revendications précédentes, **caractérisé en ce que** le besoin en courant de consigne (1) est associé à une combinaison dédiée d'éléments influençant un couple d'une chaîne cinématique du dispositif médical.

7. Dispositif médical selon l'une des revendications précédentes, **caractérisé en ce que** le besoin en courant de consigne (1) mémorisé est mémorisé en tant que données dans une mémoire de l'appareil de commande.

8. Dispositif médical selon l'une des revendications précédentes, **caractérisé en ce que** l'appareil de commande est réalisé, lorsque le besoin en courant minimal (7) dépasse une valeur seuil (8), pour déclencher un signal contenant des informations concernant l'usure du dispositif médical, lequel signal est déterminé pour un utilisateur du dispositif médical.
